# EUROPÄISCHE PATENTSCHRIFT

(11) **EP 0 578 165 B1**
(45) Veröffentlichungstag und Bekanntmachung des Hinweises auf die Patenterteilung: **05.06.1996**
(21) Anmeldenummer: 93110713.0
(22) Anmeldetag: 05.07.1993
(51) Int. Cl.: C07C 63/68, C07C 63/70, C07C 51/00, C07D 307/89

(54) **Verfahren zur Herstellung von Tetrafluorphthalsäure und/oder Tetrafluorphthalsäureanhydrid**
Process for the preparation of tetrafluorophthalic acid and/or tetrafluorophthalic anhydride
Procédé pour la préparation de l'acide tétrafluorophtalique et/ou l'anhydride tétrafluorophtalique

(30) Priorität: 10.07.1992 DE 4222719
(43) Veröffentlichungstag der Anmeldung: 12.01.1994
(73) Patentinhaber: HOECHST AKTIENGESELLSCHAFT, D-65926 Frankfurt am Main (DE)
(72) Erfinder: Pfirmann, Ralf, Dr., D-64347 Griesheim (DE); Papenfuhs, Theodor, Dr., D-60433 Frankfurt am Main (DE)

(56) Entgegenhaltungen:
- EP-A- 0 259 663
- EP-A- 0 510 490

## Beschreibung

Die vorliegende Erfindung betrifft ein Verfahren zur Herstellung von Tetrafluorphthalsäure und/oder Tetrafluorphthalsäureanhydrid.

Tetrafluorphthalsäure stellt ebenso wie Tetrafluorphthalsäureanhydrid, das sich auf einfache Weise in Tetrafluorphthalsäure umwandeln läßt, ein sehr bedeutendes Vorprodukt für die Herstellung von antibakteriellen Mitteln (DE-OS 3 318 145, EP 0 309 789, EP 0 424 850, EP 0 424 851 und EP 0 271 275) dar. Darüber hinaus spielt Tetrafluorphthalsäure oder Tetrafluorphthalsäureanhydrid bei der Herstellung von Polymeren (JP 02/29406), aber auch bei der Herstellung von Flüssigkristallen oder fotosensitiven Materialien (JP 01/268662, JP 11955 (1986)), die vorteilhafte Eigenschaften aufweisen, eine wichtige Rolle.

In der Literatur sind verschiedene Wege zur Herstellung von Tetrafluorphthalsäure beschrieben.

Tetrafluorphthalsäure kann beispielsweise aus Tetrachlorphthaloylchlorid (G.G. Yakobson et al., Zh. Obshsh. Khim. 36 (1966), 139; EP O 140 482, GB 2 146 635), aus Tetrachloranthranilsäure (S. Hayashi et al., Bull. Chem. Soc. Jap. 45 (1972), 2909), aus 1,2,3,4-Tetrafluorbenzol (L. J. Belf et al., Tetrahedron 23 (1967), 4719; Z. Naturforsch. 31B (1976), 1667), aus Tetrachlorphthalsäureanhydrid (DE-OS 3 810 093; EP O 218 111) oder aus Tetrachlorphthalodinitril (GB 2 134 900) über teilweise aufwendige und/oder technisch nicht oder nur schwer zu verwirklichende Schritte hergestellt werden. Dieselbe Aussage trifft auch für die Herstellung von Tetrafluorphthalsäure aus 1,2-Dibromtetrafluorbenzol (C.Tamborski et al., J.Organometallic Chem., 10 (1967), 385) und die von P. Sartori et al. (Chem. Ber. 101 (1968), 2004) beschriebene Methode, ausgehend von Octafluornaphthalin, zu. Ebenfalls werden N-kohlenstoffsubstituierte Tetrachlorphthalimide (EP O 259 663) eingesetzt. Nach Fluorierung können diese über teilweise unselektive Schritte (JP 02/145 538) ohne Zwischenisolierung der Tetrafluorphthalsäure, jedoch unter Isolierung eines ihrer funktionellen Derivate, zu 2,3,4,5-Tetrafluorbenzoesäure, einem ebenfalls für die Synthese von antibakteriellen Mitteln wichtigen Vorprodukt, umgesetzt werden. Die zwischenisolierten funktionellen Derivate können zu Tetrafluorphthalsäure hydrolysiert werden.

Wie die vorangegangenen Ausführungen zeigen, hat es in der Vergangenheit nicht an Versuchen gefehlt, Verfahren zur Herstellung von Tetrafluorphthalsäure zu entwickeln. Es zeigt sich aber auch, daß die Verfahren des Standes der Technik entweder von nur sehr schwer zugänglichen Einsatzstoffen ausgehen oder sich nur mit großem Aufwand realisieren lassen und zudem Wünsche hinsichtlich des Umsetzungsgrades und der Selektivität der Reaktion offenlassen.

Es besteht daher ein Bedarf nach einem Verfahren, das einerseits von relativ leicht zugänglichen Einsatzstoffen ausgeht und zum anderen sich mit einem möglichst geringen technischen Aufwand realisieren läßt und darüber hinaus den Bedarf an Hilfsstoffen und den Anfall von Abfallprodukten gering hält.

Gelöst wird diese Aufgabe durch ein Verfahren zur Herstellung von Tetrafluorphthalsäure und/oder Tetrafluorphthalsäureanhydrid. Es ist dadurch gekennzeichnet, daß man eine Verbindung der allgemeinen Formel 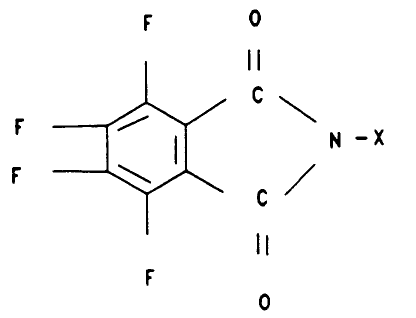
in welcher X für einen gegebenenfalls am aromatischen Kern einfach oder mehrfach durch Fluor und/ oder Chlor und/ oder Alkylgruppen mit 1 bis 4 Kohlenstoffatomen substituierten Rest 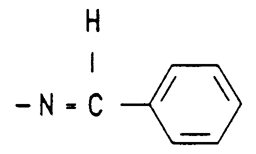
oder einen Rest 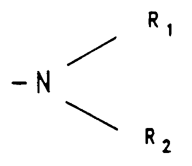
steht, worin R₁ und R₂ gleich oder verschieden sind und ein Wasserstoffatom, eine Alkylgruppe mit 1 bis 10 Kohlenstoffatomen, eine Alkyl-CO-Gruppe mit 1 bis 6 Kohlenstoffatomen im Alkylrest, eine gegebenenfalls am aromatischen Kern einfach oder mehrfach durch Fluor und/ oder Chlor und/ oder Alkylgruppen mit 1 bis 4 Kohlenstoffatomen substituierte Arylgruppe oder Aryl-CO-Gruppe bedeuten oder R₁ und R₂ zusammen einen Rest der allgemeinen Formel 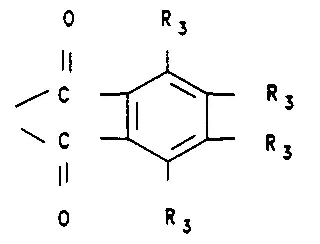
bilden, worin R₃ für ein Wasserstoffatom, ein Chloratom oder ein Fluoratom steht, mit Wasser bei 20 bis 140°C umsetzt, anschließend noch vorhandenes Wasser durch Azeotropdestillation entfernt oder die Tetrafluorphthalsäure und/ oder ihr Anhydrid mit einem wasserunlöslichen Lösungsmittel oder Lösungsmittelgemisch extrahiert.

Bei der Verbindung der vorstehend genannten allgemeinen Formel handelt es sich um N'-substituierte N-Aminotetrafluorphthalimide, die sich z. B. ausgehend von Tetrachlorphthalsäureanhydrid durch Umsetzung in Schwefelsäure mit Hydrazinsulfat und nachfolgenden Chlor-Fluor-Austausch auf vergleichsweise einfache Art herstellen lassen.

Geeignete N'-substituierte N-Aminotetrafluorphthalimide sind N'-Dialkylaminotetrafluorphthalimide, N'-Diacylaminotetrafluorphthalimide, 2,3,4,5-Tetrafluorbisphthalimide, Octafluorbisphthalimid, N'-Acylalkylaminotetrafluorphthalimide, substituierte N'-Benzylidenaminotetrafluorphthalimide, insbesondere N'-Dimethyl-aminotetrafluorphthalimid, 2,3,4,5-Tetrafluorbisphthalimid, Octafluorbisphthalimid, N'-Methylbenzoyltetrafluorphthalimid, N'-Benzylidenaminotetrafluorbisphthalimid, bevorzugt N'-Benzylidenaminotetrafluorbisphthalimid, N'-Diacetylaminotetrafluorbisphthalimid, Octafluorbisphthalimid.

Das erfindungsgemäße Verfahren läßt sich besonders vorteilhaft unter Einsatz von Octafluorbisphthalimid durchführen. Bei der Hydrolyse von einem Mol Octafluorbisphthalimid entstehen 2 Mol Tetrafluorphthalsäure, die gegebenenfalls in Tetrafluorphthalsäureanhydrid überführt werden können. Dies gestaltet die Reaktion besonders einfach und führt auch zur Vermeidung weiterer, aus der Hydrolyse entstammender Nebenprodukte.

Die Umsetzung läßt sich ohne großen Aufwand unter Verwendung von Wasser durchführen, wobei auf einen Zusatz von Hilfsstoffen, die die Umsetzung katalysieren, verzichtet werden kann. Üblicherweise setzt man 10 bis 10 000, insbesondere 100 bis 1000, bevorzugt 100 bis 600 Gew.-% Wasser, bezogen auf die als Einsatzstoff verwendete Verbindung, d.h. bezogen auf das N'-substituierte N-Aminotetrafluorphthalimid, gegebenenfalls mit einem inerten Lösungsmittel ein. Verwendet man ein inertes Lösungsmittel, so empfiehlt es sich, 100 bis 1000 Gew.-% Wasser, bezogen auf Einsatzstoff, zu verwenden.

Es empfiehlt sich, Wasser zumindest in der stöchiometrisch erforderlichen Menge oder vorteilhafterweise in einem stöchiometrischen Überschuß einzusetzen.

Ein weiterer Vorteil des erfindungsgemäßen Verfahrens besteht darin, daß auf die Verwendung üblicher saurer Katalysatoren, insbesondere Mineralsäuren, verzichtet werden kann. Dies ist überraschend, da Phthalimide im allgemeinen stabil sind und sich üblicherweise nur in Anwesenheit von Mineralsäuren hydrolysieren lassen (s. auch oben Houben-Weyl-Müller: Methoden der Organischen Chemie, Bd. VII (1952), 432-433; ibid. Bd. E V (1985), 257-263; T. Kojimoto, J. Tsuji, J. Org. Chem. 48 (1983), 1685).

Ein Grund für dieses ungewöhnliche, überraschende Verhalten könnte möglicherweise darin bestehen, daß die Umsetzung autokatalytisch abläuft. Es wäre denkbar, daß bereits Spuren von Tetrafluorphthalsäure genügen, die weitere Hydrolyse der Phthalimide schon bei ungewöhnlich niedrigen Temperaturen zu katalysieren.

Es ist möglich, zur Unterstützung der Umsetzung, Tetrafluorphthalsäure zu Beginn der Hydrolyse zuzusetzen. Beabsichtigt man, die Umsetzung mittels Tetrafluorphthalsäure zu unterstützen, so verwendet man 0,1 bis 2,5 Gew.-% Tetrafluorphthalsäure, bezogen auf die als Einsatzstoff verwendete Verbindung. Dieser Zusatz ist allerdings nicht zwingend erforderlich. Man kann in diesem Zusammenhang auf den Gebrauch von Tetrafluorphthalsäure auch verzichten.

Es ist allerdings auch möglich, Mineralsäuren als Hydrolysekatalysator einzusetzen. Dadurch wird aber die Korrosion, die durch den im Verlauf der Umsetzung durch Nebenreaktionen gebildeten Fluorwasserstoff verursacht wird, zusätzlich verstärkt.

Ein weiteres Charakteristikum der erfindungsgemäßen Umsetzung ist, daß die Hydrolyse schon bei relativ niedrigen Temperaturen abzulaufen beginnt. Die Umsetzung wird bei 20 bis 140, insbesondere 40 bis 110, bevorzugt 60 bis 100°C durch geführt. Die Umsetzung läßt sich auch bei niedrigeren Temperaturen durchführen, wobei man jedoch infolge der gegebenenfalls herabgesetzten Reaktionsgeschwindigkeit entsprechend lange Reaktionszeiten in Kauf nehmen muß.

Man setzt die als Einsatzstoff verwendete Verbindung der vorstehend genannten allgemeinen Formel zusammen mit Wasser und gegebenenfalls organischen Solventien in die Umsetzung ein. Das dabei resultierende wäßrige Gemisch weist zu Beginn der Umsetzung üblicherweise einen pH-Wert von 2 bis 8, insbesondere 4 bis 7, bevorzugt 6 bis 6,9 auf.
Im Verlauf der Reaktion kann sich der pH-Wert ändern und zu niedrigeren Werten führen, im allgemeinen durch die Bildung von Tetrafluorphthalsäure und gegebenenfalls durch Abspaltung von Fluorwasserstoff verursacht.

Es kann ebenfalls sinnvoll sein, in Anwesenheit von geringen Mengen polar aprotischer Lösungsmittel zu arbeiten, wie diese z.B. in den aus der Chlor/Fluor-Austausch-Reaktion erhaltenen Rohprodukten der N'-substituierten N-Aminotetrafluorphthalimide enthalten sind. Diese Zusätze führen, möglicherweise aufgrund ihrer Eigenschaft als Lösungsvermittler, zu höheren Reaktionsgeschwindigkeiten.

Als polar aprotische Lösungsmittel kommen Sulfolan (Tetramethylensulfon) Tetramethylensulfoxid, N,N-Diethylacetamid, N,N-Dimethylacetamid, N,N-Dimethylformamid, N-Methylpyrrolidon, Dimethylsulfoxid, Dimethylsulfon, Diphenylsulfoxid, Diphenylsulfon, Tetramethylharnstoff, Tetra-n-butylharnstoff, 1,3-Dimethylimidazolidin-2-on oder Mischungen derselben in Frage. Diese Lösungsmittel sind gegebenenfalls in der erfindungsgemäßen Reaktionmischung in Mengen zwischen etwa 0.5 % und etwa 10 %, bevorzugt zwischen etwa 1 % und etwa 2 % enthalten.

Im allgemeinen ist die Umsetzung, in Abhängigkeit von der Größe des Ansatzes und den gewählten Reaktionsbedingungen, nach etwa 4 bis etwa 24 Stunden beendet. Häufig sind Reaktionszeiten zwischen etwa 8 und etwa 12 Stunden ausreichend, nach dieser Zeit wird vom unlöslichen Rückstand filtriert. Zusatz von Klärhilfsmitteln wie Aktivkohle oder Silicaten, eingesetzt in Mengen bis etwa 10 Massen-% des eingesetzten Ausgangsmaterials und/oder fluoridfangenden Mitteln wie Calciumsalzen, Siliciumdioxid und/oder einer Siliciumdioxid enthaltenden Substanz, beispielsweise Kieselsäure, kann erforderlich sein. Als Calciumsalze sind zum Beispiel Calciumchlorid, Calciumsulfat und Calciumcarbonat zu verwenden. Siliciumdioxid kann als Aerosil^{TR}, Kieselsäure oder Quarz eingesetzt werden. Diese Zusätze werden in Mengen bis etwa 1 Mol-%, bezogen auf die als Einsatzstoff eingesetzte Verbindung der vorstehend genannten allgemeinen Formel, verwendet.

Zur Aufarbeitung der Reaktionsmischung wird gegebenenfalls - bei Einsatz von Octafluorbisphthalimid - zunächst bei pH 2 bis 3 und im allgemeinen bei Temperaturen unter etwa 50 °C das entstandene Hydrazinsalz zu elementarem Stickstoff umgesetzt. Dies geschieht durch Zusatz eines Oxidationsmittel wie Chlorbleichlauge, Natrium- oder Kaliumnitrit oder Wasserstoffperoxid. Zur Beschleunigung der Umsetzung kann es sinnvoll sein, die Zersetzung des Hydrazinsalzes durch langsame Dosierung des Oxidationsmittels während der Hydrolyse durchzuführen. Es ist jedoch auch möglich, das Oxidationsmittel nach Abschluß der Hydrolyse zuzusetzen.

Danach destilliert man entweder das Wasser azeotrop oder extrahiert die wäßrige Lösung mit organischen Lösungsmitteln.

Zur destillativen Abtrennung von Wasser verwendet man ein zur Azeotropdestillation von Wasser geeignetes Lösungsmittel, wie Toluol, Xylol, Chlortoluol, Dichlorbenzol, Chloroform, Dichlormethan, aliphatische Kohlenwasserstoffe mit 5 bis 10 Kohlenstoffatomen, beispielsweise Hexan oder Cyclohexan, und entfernt noch vorhandenes Wasser mittels einer Azeotropdestillation. Hierbei erhält man, in Abhängigkeit von den gewählten Verfahrensparametern, üblicherweise nicht Tetrafluorphthalsäure, sondern Tetrafluorphthalsäureanhydrid.

Zur Extraktion von Tetrafluorphthalsäure können als organische Lösungsmittel Dialkylether mit 1 bis 10 Kohlenstoffatomen im Alkylrest, Essigsäurealkylester mit 1 bis 10 Kohlenstoffatomen im Alkylrest, Essigsäure-(3-methoxybutyl)-ester oder andere, von der Polarität geeignete Lösungsmittel dienen. Besonders vorteilhaft kann es sein, diese Extraktion mit Trialkylaminen mit 4 bis 20 Kohlenstoffatomen je Alkylrest, bevorzugt Trialkylaminen mit 6 bis 14 Kohlenstoffatomen je Alkylrest oder Mischungen derselben, gegebenenfalls in Anwesenheit eines inerten organischen Lösungsmittels, vorzunehmen, die in der wäßrigen Mutterlauge nicht löslich sind. Wie literaturbekannt (DE 3 627 653) ist, eignen sich diese Amine zur restlosen Extraktion von sauren Verbindungen, wie Phenolen, aus verdünnten wäßrigen Lösungen. Die Amine werden erfindungsgemäß in Mengen zwischen etwa 100 Mol-% und etwa 1000 Mol-%, bevorzugt zwischen 150 Mol-% und etwa 300 Mol-%, bezogen auf die Menge der zu extrahierenden Tetrafluorphthalsäure eingesetzt. Bevorzugt ist die Verwendung von Hostarex-Marken der Hoechst AG, insbesondere der Marken A 324 und A 327, die Gemische solcher Amine darstellen. Analoge Verwendung anderer Amine, insbesondere von heterocyclischen Basen, wie Lutidinen, Collidinen oder Chinolinen kann vorteilhaft sein.

Die Menge der zu extrahierenden Tetrafluorphthalsäure wird in der Praxis sinnvollerweise über die eingesetzte Menge der Verbindung der allgemeinen Formel (N'-substituierte N-Aminotetrafluorphthalimide) abgeschätzt. Zur besseren Handhabung dieser Isolierungsvariante verdünnt man die Amine im allgemeinen mit inerten organischen Lösungsmittel, wie Toluol, Xylol, chlorierten aliphatischen und aromatischen Kohlenwasserstoffen, beispielsweise Dichlormethan, Chloroform, 1,2-Dichlorethan, Chlorbenzol, Chlortoluol, Dichlorbenzol oder ähnlichen Verbindungen, es ist aber auch möglich, ohne Verdünnungsmittel zu arbeiten. Die inerten Lösungsmittel verwendet man üblicherweise in Anteilen zwischen 20 und 1000, insbesondere zwischen 100 und 300 Gew.-%, bezogen auf eingesetzes Amin.

Dieses Verfahren besitzt den Vorteil, daß man nach Phasentrennung die organische Phase auf Temperaturen zwischen etwa 80 °C und etwa 180 °C, bevorzugt zwischen etwa 120 °C und etwa 150 °C, erhitzen kann, wobei unter Decarboxylierung 2,3,4,5-Tetrafluorbenzoesäure gebildet wird. Derselbe Effekt kann auch erzielt werden, indem man die Tetrafluorphthalsäure - wie zuvor beschrieben - extrahiert, als Rohprodukt isoliert und danach in tertiären Aminen decarboxyliert (JP 01/25 737; JP 63/295 529). Diese Variante besitzt den Vorteil, daß die erfindungsgemäß bevorzugten Amingemische preisgünstig sind und sich gut recyclisieren lassen.

Es kann während der Aufarbeitung des Reaktionsproduktes oder zur Vermeidung verfahrenstechnischer Probleme erforderlich sein, die Reaktionsmischung durch Zusatz von organischen oder anorganischen Säuren, wie Trifluormethansulfonsäure, Trifluoressigsäure, Hexafluorpropansulfonsäure, Phosphorsäure, Salpetersäure, Schwefelsäure oder Salzsäure, anzusäuern, wobei bereits geringe Mengen dieser Säuren genügen, da pH-Werte zwischen 1 und 1,5 für diese Zwecke ausreichend sind. Die nach Entfernen aller Lösungsmittel verbleibende Schmelze wird im Vakuum fraktioniert, wobei gegebenenfalls verwendete Hilfsstoffe, wie Benzaldehyde, zum Teil zurückgewonnen werden können. Aus dem übergehenden Tetrafluorphthalsäureanhydrid kann sehr leicht durch Ausrühren aus Wasser oder verdünnter Mineralsäure Tetrafluorphthalsäure erhalten werden, wie aus der EP 253 663 B1, Beispiel 3 bekannt ist. Umgekehrt verliert Tetrafluorphthalsäure schon bei Temperaturen um etwa 90 °C Wasser und wandelt sich dabei in ihr Anhydrid um, wie dies bei anderen Halogenphthalsäuren, zum Beispiel bei Tetrachlorphthalsäure bzw. deren Anhydrid (T.G. Delbridge, American Chemical Journal 41 (1909), 393) der Fall ist.

Die einzelnen Verfahrensschritte können, je nach Erfordernis, bei Atmosphärendruck, Unterdruck ober Überdruck durchgeführt werden, wobei üblicherweise die Arbeitsweise bei Atmosphärendruck bevorzugt wird.

Die folgenden Beispiele erläutern das Verfahren, ohne es zu beschränken.

### Beispiel 1

106,3 g (Reingehalt ca. 82 %) rohes Octafluorbisphthalimid werden in 300 g Wasser suspendiert und die Lösung wird mit 3 g Aktivkohle und 3 g Calciumchlorid versetzt. Die Suspension wird 16 Stunden bei 95 °C gerührt. Nach Abkühlen wird der in der anfallenden Suspension enthaltene Feststoff durch Filtration abgetrennt und das Filtrat mit Chlorbleichlauge versetzt, bis überschüssiges Chlor in der Lösung nachzuweisen ist. Die resultierende Mutterlauge wird durch Zugabe von Salzsäure auf pH 1 gestellt. Durch Extraktion der wäßrigen Phase mit Methyl-tert.-butylether (MTBE), Trocknen der organischen Phase über MgSO₄, Filtration und Entfernen des Lösungsmittels erhält man 80,4 g (338 mMol, 85 % d. Th.) Tetrafluorphthalsäure, Kristallisation aus 20%iger Salzsäure liefert 75,0 g (315 mMol, 79 % d. Th.) Tetrafluorphthalsäure (Schmelzpunkt 155-157°C).

### Beispiel 2

500 g (Reingehalt 340 g, ca. 50 g N,N-Dimethylacetamid) rohes Octafluorbisphthalimid (aus Chlor/Fluor-Austauschreaktion) werden in 2000 g Wasser suspendiert, und die Lösung wird mit 30 g Aktivkohle und 50 g Kieselsäure versetzt. Die Suspension wird 8 Stunden bei 100°C gerührt. Nach Abkühlen wird der in der anfallenden Suspension enthaltene Feststoff durch Filtration abgetrennt und das Filtrat durch Zugabe von Trifluoressigsäure auf pH 1 gestellt. Durch Extraktion der wäßrigen Phase mit Di-n-butylether, Trocknen der organischen Phase über MgSO₄, Filtration und Entfernen des Lösungsmittels erhält man 321,9 g (1,35 Mol, 85 % d. Th.) Tetrafluorphthalsäure, Kristallisation aus 20%iger Salzsäure liefert 300 g (1,26 Mol, 79 % d. Th.) Tetrafluorphthalsäure (Schmelzpunkt 155-157°C).

### Beispiel 3

100 g (Reingehalt 71 g, ca. 10 g N-Methylpyrrolidon) rohes N'-Dimethylamino-N-aminotetrafluorphthalimid (X = Dimethylamino-) werden in 600 g Wasser suspendiert und die Lösung mit 8 g Aktivkohle und 5 g Calciumsulfat versetzt. Die Suspension wird 20 Stunden bei 85°C gerührt. Nach Abkühlen wird der in der anfallenden Suspension enthaltene Feststoff durch Filtration abgetrennt und das Filtrat durch Zugabe von Phosphorsäure auf pH 1,5 gestellt. Durch Extraktion der wäßrigen Phase mit Butoxyl^{TR} (Essigsäure-(3-methoxybutyl)-ester), Trocknen über MgSO₄, Filtration und Entfernen des Lösungsmittels erhält man 110,8 g (466 mMol, 86 % d. Th.) Tetrafluorphthalsäure, Kristallisation aus 20%iger Salzsäure liefert 95,9 g (403 mMol, 75 % d. Th.) Tetrafluorphthalsäure (Schmelzpunkt 154-157°C).

### Beispiel 4

50 g (Reingehalt 33 g, ca. 8 g Sulfolan) rohes N'-Benzylidenaminotetrafluorphthalimid (X = Benzylidenamino-) werden in 600 g Wasser suspendiert, und die Lösung wird mit 4 g Perlite und 2 g Aerosil versetzt. Die Suspension wird 16 Stunden bei 100°C gerührt. Nach Abkühlen wird der in der Suspension enthaltene Feststoff abgetrennt und das Filtrat durch Zugabe von Schwefelsäure auf pH 1 gestellt. Durch Extraktion der wäßrigen Phase mit Essigsäureethylester, Trocknen über MgSO₄, Filtration und Entfernen des Lösungsmittels und des enthaltenen Benzaldehydes erhält man 35 g (148 mMol, 72 % d. Th.) Tetrafluorphthalsäure (Reingehalt mittels high performance liquid chromatography (HPLC) bestimmt).

### Beispiel 5

100 g rohes Octafluorbisphthalimid (Reingehalt mittels gas chromatography (GC) bestimmt: 75 %, ca. 8 g Restgehalt N,N-Dimethylacetamid) werden mit 10 g Wasser in 500 g Xylol suspendiert, und die beim Erhitzen entstehende Lösung wird 72 Stunden bei 100°C gerührt. Es wird heiß filtriert und das Filtrat unter Rühren 5 Stunden bei 0°C gehalten. Das ausfallende gelbgefärbte Tetrafluorphthalsäureanhydrid wird durch Filtration isoliert. Durch Trocknen erhält man 59,8 g (270 mMol, 79 % (roh)) Tetrafluorphthalsäureanhydrid, wobei die Mutterlauge noch geringe Mengen Produkt (1 bis 3 %) enthält und für weitere Ansätze erneut verwendet wird.

### Beispiel 6

109,0 g (250 mMol) Octafluorbisphthalimid (aus Essigsäureethylester kristallisiert, Schmp. 302,8°C, bestimmt mittels differential scanning calorimetry (DSC)) werden in 400 g Wasser suspendiert und die Lösung wird mit 2 g Aktivkohle und 5 g Calciumchlorid versetzt. Die Suspension wird 18 Stunden bei 90°C gerührt. Nach Abkühlen wird der in der anfallenden Suspension enthaltene Feststoff durch Filtration abgetrennt. Durch Extraktion der wäßrigen Phase mittels Diisopropylether, Trocknen über MgSO₄, Filtration und Entfernen des Lösungsmittels erhält man 108,3 g (460 mMol, 91 %) Tetrafluorphthalsäure (Gesamtausbeute, roh 86 %).

### Beispiel 7

200 g rohes (Octafluorbisphthalimid (Reingehalt 86 %, 172 g, 394 mMol) werden in 1200 g Wasser suspendiert und mit 50 g Aktivkohle und 50 g Kieselsäure versetzt. Die Suspension wird 10 Stunden bei 95°C gerührt. 2 Stunden nach Beginn der Umsetzung tropft man innerhalb von 5 Stunden eine Lösung von 63,3 g (920 mMol) Natriumnitrit in 200 g Wasser zu. Nach Abkühlen wird eine braungefärbte Suspension erhalten, die mittels Schwefelsäure auf pH 1 eingestellt wird. Durch Filtration werden die festen Bestandteile abgetrennt, und der Filterkuchen wird zweimal mit je 100 ml Wasser gewaschen. Zur wäßrigen Phase werden 400 g Xylol zugegeben und danach unter Rühren 200 g Hostarex A 324 zulaufen gelassen. Man rührt 0.5 Stunden kräftig, trennt die organische Phase, die Tetrafluorphthalsäure enthält, ab und verwirft die wäßrige Phase. Die organische Phase wird 4 Stunden zum Sieden erhitzt, wobei starke Gasentwicklung auftritt. Die braune Lösung wird zweimal mit verdünnter Kalilauge ausgerührt, die organische Phase kann wieder zur Extraktion verwendet werden. Die alkalische Lösung wird mit Schwefelsäure (96 %) auf pH 1 eingestellt und mit MTBE extrahiert. Nach Trocknen und Entfernen des Lösungsmittels werden 107,4 g (553 mMol, 70 % d. Th.) 2,3,4,5-Tetrafluorbenzoesäure erhalten.

### Beispiel 8

Zu 150 g rohem Octafluorbisphthalimid (Reingehalt 60 %) in 250 g Wasser gibt man 5 g gekörnte Aktivkohle, 10 g Perlite und 15 g Quarz. Diese Mischung wird 8 Stunden bei 100°C gerührt und nach Abkühlen mittels 36%iger Salzsäure auf pH 2 eingestellt. Man dosiert anschließend Chlorbleichlauge (13%) unter Rühren unter die Flüssigkeitsoberfläche (Gasentwicklung), bis die Jod-Stärke-Probe einen Überschuß an Oxidationsmittel anzeigt. Bei 30°C werden die Feststoffe abfiltriert und der Filterkuchen zweimal mit 50 g Wasser gewaschen. Die auf pH 1.5 eingestellte Mutterlauge wird mit 200 g 1,2 Dichlorbenzol/150 g Hostarex A 327 extrahiert. Die weitere Verarbeitung erfolgt, wie in Beispiel 7 angegeben. Auf diese Weise werden nach Umlösung in Wasser 52,1 g (268 mMol, 65 % d. Th.) 2,3,4,5-Tetrafluorbenzoesäure (Schmelzpunkt 85,6°-87,5°C) als farbloser Festkörper erhalten.

### Beispiel 9

350 g rohe Tetrafluorphthalsäure werden mit 800 g Xylol am Wasserabscheider erhitzt, bis kein Wasser mehr übergeht (5 Stunden). Man läßt die resultierende Suspension abkühlen und filtriert das ausfallende Anhydrid ab. Man erhält 301 g Tetrafluorphthalsäureanhydrid, wobei die Mutterlauge, die noch ca. 5 g Tetrafluorphthalsäureanhydrid enthält, für weitere Entwässerungen verwendet wird. Das Tetrafluorphthalsäureanhydrid wird anschließend durch Fraktionierung gereinigt, wobei es (bei 1 mbar/95°C-105°C) als farblose Flüssigkeit übergeht und als farblose, kristalline Masse vom Schmp. 93-94°C erstarrt. Auf diese Weise erhält man 255 g (1,16 Mol) Tetrafluorphthalsäureanhydrid mit einem Reingehalt von > 99 % (GC, HPLC).

## Patentansprüche

1. Verfahren zur Herstellung von Tetrafluorphthalsäure und/ oder Tetrafluorphthalsäureanhydrid, dadurch gekennzeichnet, daß man eine Verbindung der allgemeinen Formel 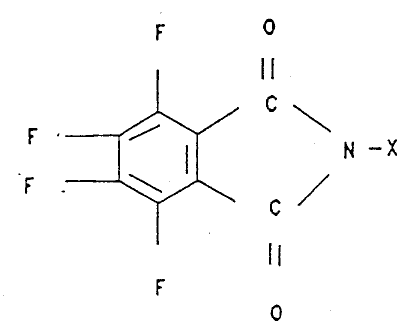 in welcher X für einen gegebenenfalls am aromatischen Kern einfach oder mehrfach durch Fluor und/ oder Chlor und/ oder Alkylgruppen mit 1 bis 4 Kohlenstoffatomen substituierten Rest 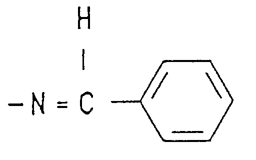 oder einen Rest 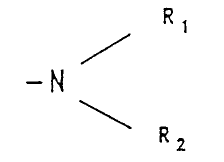 steht, worin R₁ und R₂ gleich oder verschieden sind und ein Wasserstoffatom, eine Alkylgruppe mit 1 bis 10 Kohlenstoffatomen, eine Alkyl-CO-Gruppe mit 1 bis 6 Kohlenstoffatomen im Alkylrest, eine gegebenenfalls am aromatischen Kern einfach oder mehrfach durch Fluor und/ oder Chlor und/ oder Alkylgruppen mit 1 bis 4 Kohlenstoffatomen substituierte Arylgruppe oder Aryl-CO-Gruppe bedeuten, oder R₁ und R₂ zusammen einen Rest der allgemeinen Formel 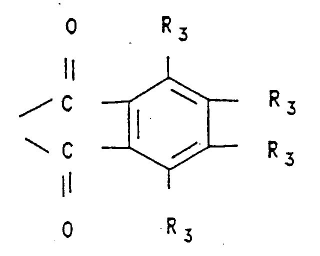 bilden, worin R³ für ein Wasserstoffatom, ein Chloratom oder ein Fluoratom steht, mit Wasser bei 20 bis 140°C umsetzt, anschließend noch vorhandenes Wasser durch Azeotropdestillation entfernt oder die Tetrafluorphthalsäure und/ oder ihr Anhydrid mit einem wasserunlöslichen Lösungsmittel oder Lösungsmittelgemisch extrahiert.

2. Verfahren nach Anspruch 1, dadurch gekennzeichnet, daß man als Verbindung Octafluorbisphthalimid der Formel 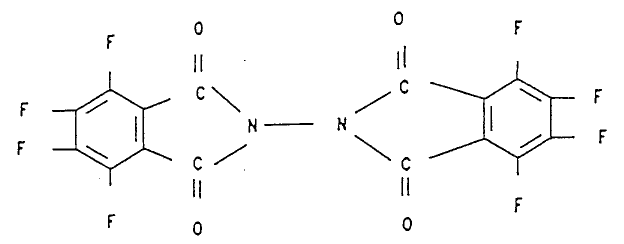 einsetzt.

3. Verfahren nach Anspruch 1 oder 2, dadurch gekennzeichnet, daß man 10 bis 10000, insbesondere 100 bis 1000, bevorzugt 100 bis 600 Gew.-% Wasser, bezogen auf die als Einsatzstoff verwendete Verbindung, gegebenenfalls mit einem inerten Lösungsmittel einsetzt.

4. Verfahren nach einem oder mehreren der Ansprüche 1 bis 3, dadurch gekennzeichnet, daß man die Umsetzung ohne Zusatz von Mineralsäuren durchführt.

5. Verfahren nach einem oder mehreren der Ansprüche 1 bis 4, dadurch gekennzeichnet, daß man zu Beginn der Umsetzung Tetrafluorphthalsäure zusetzt.

6. Verfahren nach einem oder mehreren der Ansprüche 1 bis 5, dadurch gekennzeichnet, daß man 0,1 bis 2,5 Gew.% Tetrafluorphthalsäure, bezogen auf die als Einsatzstoff verwendete Verbindung, zusetzt.

7. Verfahren nach einem oder mehreren der Ansprüche 1 bis 6, dadurch gekennzeichnet, daß man die Umsetzung bei 40 bis 110, bevorzugt 60 bis 100 °C durchführt.

8. Verfahren nach einem oder mehreren der Ansprüche 1 bis 7, dadurch gekennzeichnet, daß das wäßrige Gemisch zu Beginn der Umsetzung einen pH-Wert von 2 bis 8, insbesondere 4 bis 7, bevorzugt 6 bis 6,9 aufweist.

9. Verfahren nach einem oder mehreren der Ansprüche 1 bis 8, dadurch gekennzeichnet, daß man die Umsetzung in Gegenwart von Calciumsalzen, Siliciumdioxid oder einer Siliciumdioxid enthaltenden Substanz, insbesondere Kieselsäure durchführt.

10. Verfahren nach einem oder mehreren der Ansprüche 1 bis 9, dadurch gekennzeichnet, daß man Aktivkohle oder Silicate als Filterhilfsmittel einsetzt.

11. Verfahren nach einem oder mehreren der Ansprüche 1 bis 10, dadurch gekennzeichnet, daß man die Tetrafluorphthalsäure und/ oder ihr Anhydrid mittels eines Dialkylethers mit 1 bis 10 Kohlenstoffatomen je Alkylrest, eines Essigsäurealkylesters mit 1 bis 10 Kohlenstoffatomen in Alkylrest, Essigsäure-(3-methoxylbutyl)-ester, eines Trialkylamins mit 4 bis 20 Kohlenstoffatomen je Alkylrest oder Mischungen dieser Amine, gegebenenfalls in Anwesenheit eines inerten organischen Lösungsmittels, wie Toluol, Xylolen, chlorierter aliphatischer und aromatischer Kohlenwasserstoffe, beispielsweise Dichlormethan, Chloroform, 1,2-Dichlorethan, Chlorbenzol, Chlortoluol, Dichlorbenzol, extrahiert.

12. Verfahren nach einem oder mehreren der Ansprüche 1 bis 11, dadurch gekennzeichnet, daß man ein zur Azeotropdestillation von Wasser geeignetes Lösungsmittel, wie Toluol, Xylol, Chlortoluol, Dichlorbenzol, Chloroform, Dichlormethan, 1,2-Dichlorethan, aliphatische Kohlenwasserstoffe mit 5 bis 10 Kohlenstoffatomen, beispielsweise Hexan oder Cyclohexan zusetzt und noch vorhandenes Wasser mittels einer Azeotropdestillation entfernt.

13. Verfahren nach einem oder mehreren der Ansprüche 1 bis 12, dadurch gekennzeichnet, daß man gegebenenfalls gebildete Hydrazinsalze mittels eines Oxidationsmittels, wie Chlorbleichlauge, Kaliumnitrit, Natriumnitrit oder Wasserstoffperoxid, zu Stickstoff oxidiert.

14. Verfahren nach einem oder mehreren der Ansprüche 1 bis 13, dadurch gekennzeichnet, daß man das Oxidationsmittel während der Umsetzung oder nach Beendigung der Umsetzung zusetzt.

15. Verfahren nach einem oder mehreren der Ansprüche 1 bis 14, dadurch gekennzeichnet, daß man nach azeotropem Entfernen des noch vorhandenen Wassers im heißen Zustand filtriert und aus der erkalteten Mutterlauge Tetrafluorphthalsäureanhyrid abfiltriert.

16. Verfahren nach einem oder mehreren der Ansprüche 1 bis 15, dadurch gekennzeichnet, daß man die in roher Form extrahierte Tetrafluorphthalsäure im Extraktionsmittel zu 2,3,4,5-Tetrafluorbenzoesäure decarboxyliert.

## Claims

1. A process for the preparation of tetrafluorophthalic acid and/or tetrafluorophthalic anhydride, which comprises reacting a compound of the formula 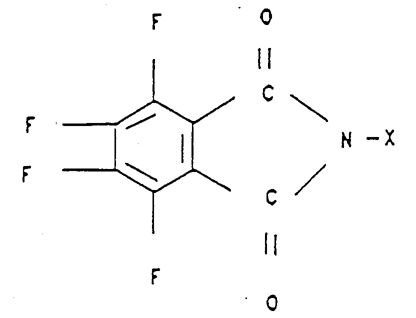 in which X is a radical 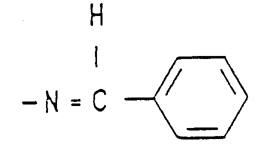 which is optionally mono- or polysubstituted on the aromatic nucleus by fluorine and/or chlorine and/or alkyl groups having 1 to 4 carbon atoms, or is a radical 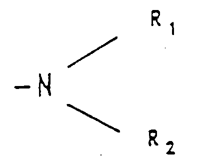 in which R₁ and R₂ are identical or different and are a hydrogen atom, an alkyl group having 1 to 10 carbon atoms, an alkyl-CO- group having 1 to 6 carbon atoms in the alkyl radical or an aryl group or aryl-CO- group which is optionally mono- or polysubstituted on the aromatic nucleus by fluorine and/or chlorine and/or alkyl groups having 1 to 4 carbon atoms, or R₁ and R₂ together form a radical of the formula 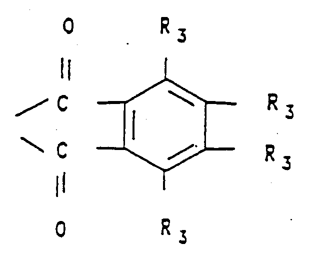 in which R₃ is a hydrogen atom, a chlorine atom or a fluorine atom, with water at 20 to 140°C, and subsequently removing the water still present by azeotropic distillation or extracting the tetrafluorophthalic acid and/or its anhydride with a water-insoluble solvent or solvent mixture.

2. The process as claimed in claim 1, wherein octafluorobisphthalimide of the formula 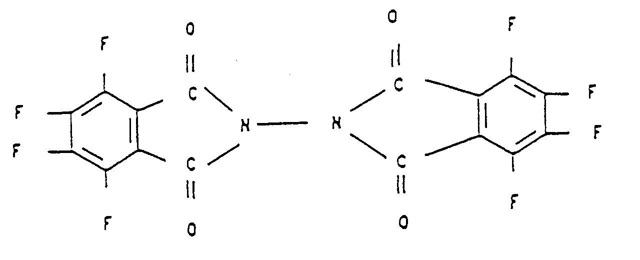 is employed as the compound.

3. The process as claimed in claim 1 or 2, wherein 10 to 10000, in particular 100 to 1000, preferably 100 to 600% by weight of water, based on the compound used as the starting substance, is employed, with or without an inert solvent.

4. The process as claimed in one or more of claims 1 to 3, wherein the reaction is carried out without addition of mineral acids.

5. The process as claimed in one or more of claims 1 to 4, wherein tetrafluorophthalic acid is added at the start of the reaction.

6. The process as claimed in one or more of claims 1 to 5, wherein 0.1 to 2.5% by weight of tetrafluorophthalic acid, based on the compound used as the starting substance, is added.

7. The process as claimed in one or more of claims 1 to 6, wherein the reaction is carried out at 40 to 110, preferably 60 to 100°C.

8. The process as claimed in one or more of claims 1 to 7, wherein the aqueous mixture has a pH of 2 to 8, in particular 4 to 7, preferably 6 to 6.9, at the start of the reaction.

9. The process as claimed in one or more of claims 1 to 8, wherein the reaction is carried out in the presence of calcium salts, silicon dioxide or a substance containing silicon dioxide, in particular silicic acid.

10. The process as claimed in one or more of claims 1 to 9, wherein active charcoal or silicates are employed as a filtration auxiliary.

11. The process as claimed in one or more of claims 1 to 10, wherein the tetrafluorophthalic acid and/or its anhydride is extracted by means of a dialkyl ether having 1 to 10 carbon atoms per alkyl radical, an alkyl acetate having 1 to 10 carbon atoms in the alkyl radical, 3-methoxybutyl acetate, a trialkylamine having 4 to 20 carbon atoms per alkyl radical or a mixture of these amines, if appropriate in the presence of an inert organic solvent, such as toluene, a xylene or a chlorinated aliphatic or aromatic hydrocarbon, for example dichloromethane, chloroform, 1,2-dichloroethane, chlorobenzene, chlorotoluene or dichlorobenzene.

12. The process as claimed in one or more of claims 1 to 11, wherein a solvent suitable for azeotropic distillation of water, such as toluene, xylene, chlorotoluene, dichlorobenzene, chloroform, dichloromethane, 1,2-dichloroethane or an aliphatic hydrocarbon having 5 to 10 carbon atoms, for example hexane or cyclohexane, is added and the water still present is removed by means of azeotropic distillation.

13. The process as claimed in one or more of claims 1 to 12, wherein any hydrazine salts formed are oxidized to nitrogen by means of an oxidizing agent, such as chlorine bleaching liquor, potassium nitrite, sodium nitrite or hydrogen peroxide.

14. The process as claimed in one or more of claims 1 to 13, wherein the oxidizing agent is added during the reaction or after the reaction has ended.

15. The process as claimed in one or more of claims 1 to 14, wherein, after azeotropic removal of the water still present, the mixture is filtered in the hot state and tetrafluorophthalic anhydride is filtered off from the cooled mother liquor.

16. The process as claimed in one or more of claims 1 to 15, wherein the tetrafluorophthalic acid extracted in crude form is decarboxylated in the extraction agent to give 2,3,4,5-tetrafluorobenzoic acid.

## Revendications

1. Procédé pour la préparation d'acide tétrafluorophtalique et/ou d'anhydride tétrafluorophtalique, caractérisé en ce que l'on fait réagir un composé de formule générale : 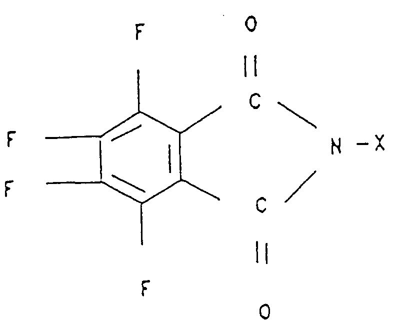 dans laquelle X représente un radical éventuellement substitué une ou plusieurs fois sur le noyau aromatique par le fluor et/ou le chlore et/ou des groupes alkyle avec 1 à 4 atomes de carbone 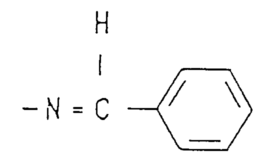 ou un radical 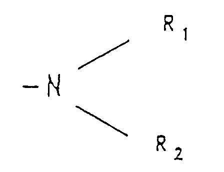 où R₁ et R₂ sont identiques ou différents et représentent un atome d'hydrogène, un groupe alkyle avec 1 à 10 atomes de carbone, un groupe alkyle-CO- avec 1 à 6 atomes de carbone dans le radical alkyle, un groupe aryle-CO- ou aryle éventuellement substitué une ou plusieurs fois sur le noyau aromatique par le fluor, et/ou le chlore et/ou des groupes alkyle avec 1 à 4 atomes de carbone, ou R₁ et R₂ ensemble forment un radical de formule générale : 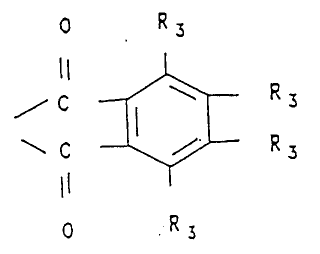 dans laquelle R₃ représente un atome d'hydrogène, un atome de chlore ou un atome de fluor, avec de l'eau à 20 à 140 °C, puis on élimine l'eau encore présente par distillation azéotropique ou on extrait l'acide tétrafluorophtalique et/ou son anhydride avec un solvant ou un mélange de solvants insolubles dans l'eau.

2. Procédé selon la revendication 1, caractérisé en ce que l'on utilise comme composé l'octafluorobisphtalimide de formule :

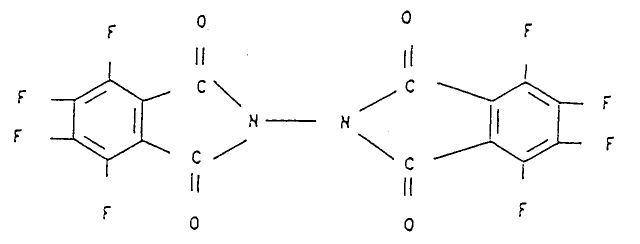

3. Procédé selon la revendication 1 ou 2, caractérisé en ce que l'on utilise de 10 à 10 000, plus particulièrement de 100 à 1000, de manière préférée de 100 à 600 % en poids d'eau par rapport au composé utilisé comme matière de départ, éventuellement avec un solvant inerte.

4. Procédé selon une ou plusieurs des revendications 1 à 3, caractérisé en ce que l'on met en oeuvre la réaction sans addition d'acides minéraux.

5. Procédé selon une ou plusieurs des revendications 1 à 4, caractérisé en ce que l'on ajoute l'acide tétrafluorophtalique au début de la réaction.

6. Procédé selon une ou plusieurs des revendications 1 à 5, caractérisé en ce que l'on ajoute de 0,1 à 2,5 % en poids d'acide tétrafluorophtalique par rapport au composé utilisé comme composé de départ.

7. Procédé selon une ou plusieurs des revendications 1 à 6, caractérisé en ce que l'on met en oeuvre la réaction à une température de 40 à 110, de préférence de 60 à 100 °C.

8. Procédé selon une ou plusieurs des revendications 1 à 7, caractérisé en ce que le mélange aqueux présente au début de la réaction un pH de 2 à 8, plus particulièrement de 4 à 7, de manière préférée de 6 à 6,9.

9. Procédé selon une ou plusieurs des revendications 1 à 8, caractérisé en ce que l'on met en oeuvre la réaction en présence de sels de calcium, de dioxyde de silicium ou d'une substance contenant du dioxyde de silicium, plus particulièrement d'acide silicique.

10. Procédé selon une ou plusieurs des revendications 1 à 9, caractérisé en ce que l'on utilise du charbon actif ou des silicates comme adjuvants de filtration.

11. Procédé selon une ou plusieurs des revendications 1 à 10, caractérisé en ce que l'on extrait l'acide tétrafluorophtalique et/ou son anhydride à l'aide d'un éther dialkylique comportant de 1 à 10 atomes de carbone par radical d'alkyle, d'un acétate d'alkyle comportant de 1 à 10 atomes de carbone dans le radial alkyle, de l'acétate de 3-méthoxybutyle, d'une trialkylamine comportant de 4 à 20 atomes de carbone par radical alkyle ou des mélanges de ces amines, éventuellement en présence d'un solvant organique inerte, comme toluène, xylène, hydrocarbures aliphatiques et aromatiques chlorés, comme par exemple dichlorométhane, chloroforme, 1,2-dichloroéthane, chlorobenzène, chlorotoluène, dichlorobenzène.

12. Procédé selon une ou plusieurs des revendications 1 à 11, caractérisé en ce que l'on ajoute un solvant approprié à la distillation azéotropique de l'eau, comme toluène, xylène, chlorotoluène, dichlorobenzène, chloroforme, dichlorométhane, 1,2-dichloroéthane, hydrocarbures aliphatiques comportant de 5 à 10 atomes de carbone, par exemple hexane ou cyclohexane et on élimine l'eau encore présente par une distillation azéotropique.

13. Procédé selon une ou plusieurs des revendications 1 à 12, caractérisé en ce que l'on oxyde les sels d'hydrazine éventuellement formés à l'aide d'un agent oxydant comme la solution d'hypochlorite de sodium, le nitrite de potassium, le nitrite de sodium ou le peroxyde d'hydrogène, jusqu'à l'azote.

14. Procédé selon une ou plusieurs des revendications 1 à 13, caractérisé en ce que l'on ajoute l'agent oxydant au cours de la réaction ou après avoir terminé la réaction.

15. Procédé selon une ou plusieurs des revendications 1 à 14, caractérisé en ce qu'après l'élimination azéotropique de l'eau encore présente, on filtre à chaud et on sépare par filtration l'anhydride tétrafluorophtalique de la liqueur-mère refroidie.

16. Procédé selon une ou plusieurs des revendications 1 à 15, caractérisé en ce que l'on décarboxyle l'acide tétrafluorophtalique, extrait sous forme brute, dans l'agent d'extraction pour obtenir l'acide 2,3,4,5-tétrafluorobenzoïque.
